(19) Europäisches Patentamt
European Patent Office
Office européen des brevets

(11) Publication number: 0 028 853
B2

# (12) NEW EUROPEAN PATENT SPECIFICATION

(45) Date of publication of the new patent specification: 02.11.88

(51) Int. Cl.⁴: A 61 K 7/32

(21) Application number: 80201014.0

(22) Date of filling: 27.10.80

(54) **Antiperspirant compositions.**

(30) Priority: 07.11.79 US 92113
30.06.80 US 163903

(43) Date of publication of application: 20.05.81 Bulletin 81/20

(45) Publication of the grant of the patent: 11.07.84 Bulletin 84/28

(45) Mention of the opposition decision: 02.11.88 Bulletin 88/44

(84) Designated Contracting States: AT BE CH DE FR GB IT LI LU NL

(56) References cited:
AU-A-510 504
DE-A-2 050 712
DE-A-2 741 819
FR-A-2 229 753
FR-A-2 269 923
FR-A-2 320 730
FR-A-2 349 326
GB-A-1 467 676
GB-A-2 018 590

Kirk-Othmer "Encyclopedia of Chemical Technology, 3rd Ed., 20, pp. 923,924,934

The file contains technical information submitted after the application was filed and not included in this specification

(73) Proprietor: THE PROCTER & GAMBLE COMPANY, 301 East Sixth Street, Cincinnati Ohio 45202 (US)

(72) Inventor: Beckmeyer, Mary Virginia, 2460 Mustang Drive, Cincinnati Ohio 45211 (US)
Inventor: Davis, James Arthur, 1140 Hollywood Avenue, Cincinnati Ohio 45224 (US)
Inventor: Kelm, Gary Robert, 8524 Althaus Road, Cincinnati Ohio 45247 (US)

(74) Representative: Brooks, Maxim Courtney, Procter & Gamble (NTC) Limited Whitley Road Longbenton, Newcastle- upon- Tyne NE12 9TS (GB)

EP 0 028 853 B2

LIBER, STOCKHOLM 1988

## Description

This invention relates to novel antiperspirant compositions which are useful in a variety of dispensing devices. The compositions comprise an antiperspirant material, a bulking/suspending agent, a volatile silicone and a non-volatile emollient.

The use of volatile silicones and non-volatiles emollients in a variety of compositions has been suggested. References disclosing such compositions include US-A-3 836 647, US-A-3 903 258, US-A-4 053 581, US-A-4 054 670, US-A-4 065 564, US-A-4 073 880, US-A-4 083 956, US-A-4 122 029 and GB-A-2 018 590, US-A-4 083 956, for example discloses anhydrous antiperspirant compositions in the form of creams wherein the antiperspirant is dispersed in a gel of liquid emollient material, a volatile silicone optionally being added as anti-syneresis agent. US-A-4 053 518 relates to antiperspirant compositions in the form of alcoholic solutions comprising mixtures of volatile and non-volatile silicones. GB-A-2 018 590 discloses antiperspirant compositions in suspension form comprising a volatile silicone and up to 4 % of a less volatile hydrophobic liquid, specifically isopropyl myristate.

Nevertheless there is no disclosure in the art of antiperspirant compositions in suspension form comprising a volatile silicone and from 1 % to 35 % of a non-volatile silicone emollient. Furthermore, the art references do not suggest the surprising improvement in antiperspirant efficacy found with the present invention.

Accordingly, it is an object of the present invention to provide antiperspirant compositions having enhanced antiperspirant efficacy.

It is further object of the present invention to provide antiperspirant compositions which are suitable for use in a number of dispensing devices.

According to the present invention there is provided an antiperspirant composition in the form of a suspension comprising:

(a) from 10 % to 70 % by weight of a particulate antiperspirant material;
(b) from 1 % to 15 % by weight of a bulking/suspending agent; and
(c) from 10 to 80 % by weight of a cyclic polydimethylsiloxane having from 3 to 6 silicon atoms and a viscosity of less than 10 $mm^2s^{-1}$ (cSt) at 25°C or a linear polydimethylsiloxane having from 3 to 9 silicon atoms and a viscosity of less than 5 $mm^2s^{-1}$ (cSt) at 25° C;

wherein the composition additionally comprises:

(d) from 1 % to 35 % by weight of a non-volatile emollient selected from silicones having a viscosity of from 10 to 100,000 $mm^2s^{-1}$ (cSt) at 25°C.

In preferred embodiments, the particulate antiperspirant material comprises from 15 % to 60 % by weight of composition, the bulking/suspending agent comprises from 2 % to 8 % by weight of composition, the cyclic or linear polydimethylsiloxane comprises from 15 % to 70 % by weight of composition, and the non-volatile emollient comprises from 5 % to 30 % by weight of composition. The compositions are preferably anhydrous (containing less than about 1.5 % water).

The necessary as well as optional components of the present compositions are described in detail below.

The present compositions contain from 10 % to 70 %, preferably 15 % to 60 %, by weight of a particulate antiperspirant material. Such materials include for example, many aluminum or zirconium astringent salts or complexes and are well known in the antiperspirant art.

Any aluminum astringent antiperspirant salt or aluminum and/or zirconium astringent complex in particulate form can be employed herein. Salts useful as astringent antiperspirant salts or as components of astringent complexes include aluminum halides, aluminum hydroxyhalides, zirconyl oxyhalides, zirconyl hydroxyhalides and mixtures of these salt materials.

Aluminum salts of this type include aluminum chloride and the aluminum hydroxyhalides having the general formula $Al_2(OH)_xQ_y.XH_2O$ where Q is chlorine, bromine or iodine; where x is 2 to 5 and $x+y=6$ and x and y do not need to be integers; and where X is about 1 to 6. Aluminum salts of this type can be prepared in the manner described more fully in US-A-3 887 692 and US-A-3 904 741.

The zirconium compounds which are useful in the present invention include both the zirconium oxy salts and zirconium hydroxy salts, also referred to as the zirconyl salts and zirconyl hydroxy salts. These compounds may be represented by the following general empirical formula:

$$ZrO(OH)_{2-nz}B_z$$

wherein z may vary from about 0.9 to 2 and need not be an integer, n is the valence of B, 2-nz is greater than or equal to 0, and B may be selected from halides, nitrate, sulfamate, sulfate and mixtures thereof. Although only zirconium compounds are exemplified in this specification, it will be understood that other Group IV B metals, including hafnium could be used in the present invention.

As with the basic aluminum compounds, it will be understood that the above formula is greatly simplified and is intended to represent and include compounds having coordinated and/or bound water in various quantitites, as well as polymers, mixtures and complexes of the above. As will be seen from the above formula, the zirconium hydroxy salts actually represent a range of compounds having various amounts of the hydroxy group, varying from about 1.1 to only

slightly greater than 0 groups per molecule.

Several types of antiperspirant complexes utilizing the above antiperspirant salts are known in the art. For example US-A-3 792 068 discloses complexes of aluminum, zirconium, and amino acids such as glycine. Complexes such as those disclosed in this patent and other similar complexes are commonly known as ZAG. ZAG complexes are chemically analyzable for the presence of aluminum, zirconium and chlorine, ZAG complexes useful herein are identified by the specification of both the molar ratio of aluminum to zirconium (hereinafter "Al:Zr" ratio) and the molar ratio of total metal to chlorine (hereinafter "Metal:Cl" ratio). ZAG complexes useful herein have an Al:Zr ratio of from 1.67 to 12.5 and a Metal:Cl ratio of from 0.73 to 1 93.

Preferred ZAG complexes are formed by

(A) Co-dissolving in water
  (1) one part $Al_2(OH)_{6-m}Q_m$, wherein Q is an anion selected from chloride, bromide and iodide and m is a number from 0.8 to 2.0;
  (2) x parts $ZrO(OH)_{2-a}Q_a \cdot nH_2O$ where Q is chloride, bromide or iodide; where a is from 1 to 2; where n is from 1 to 8; and where x has a value of from 0.16 to 1.2;
  (3) p parts neutral amino acid selected from glycine, dl-tryptophane, dl-β-phenylalanine, dl-valine, dl-methionine, and β-alanine, and where p has a value of from 0.06 to 0.53;
(B) Co-drying the resultant mixture to a friable solid; and
(C) Reducing the resultant dried inorganic-organic antiperspirant complex to particulate form.

A preferred aluminum compound for preparation of such ZAG type complexes is aluminum chlorhydroxide of the empirical formula $Al_2(OH)_5Cl \cdot 2H_2O$. Preferred zirconium compounds for preparation of such ZAG-type complexes are zirconylhydroxychloride having the empirical formula $ZrO(OH)Cl \cdot 3H_2O$ and the zirconyl hydroxyhalides of the empirical formula $ZrO(OH)_{2-a}Cl_2 \cdot nH_2O$ wherein a is from 1.5 to 1.87 and n is from 1 to 7. The preferred amino acid for preparing such ZAG-type complexes is glycine of the formula $CH_2(NH_2)COOH$. Salts of such amino acids can also be employed in such antiperspirant complexes, see US-A-4 017 599.

A wide variety of other types of antiperspirant complexes are also known in the art. For example, US-A-3 903 258 discloses a zirconium aluminum complex prepared by reacting zirconyl chloride with aluminum hydroxide and aluminum chlorhydroxide. US-A-3 979 510 discloses an antiperspirant complex formed from certain aluminum compounds, certain zirconium compounds and certain complex aluminum buffers. US-A-3 981 896 discloses an antiperspirant complex prepared from an aluminum polyol compound, a zirconium compound and an organic buffer. US-A-3 970 748 discloses an aluminum chlorhydroxy glycinate complex of the approximate general formula $[Al_2(OH_4)Cl][H_2CNH_2COOH]$.

Of all the above types of antiperspirant actives, preferred compounds include the 5/6 basic aluminum salts of the empirical formula $Al_2(OH)_5Cl \cdot 2H_2O$; mixtures of $AlCl_3 \cdot 6H_2O$ and $Al_2(OH)_5Cl \cdot 2H_2O$ with aluminum chloride to aluminum hydroxychloride weight ratios of up to 0.5; ZAG type complexes wherein the zirconium salt is $ZrO(OH)Cl \cdot 3H_2O$; the aluminum salt is $Al_2(OH)_5Cl \cdot 2H_2O$ or the aforementioned mixtures of $AlCl_3 \cdot 6H_2O$ and $Al_2(OH)_5Cl \cdot 2H_2O$ wherein the total metal to chloride molar ratio in the complex is less than 1.25 and the Al:Zr molar ratio is about 3.3; and the amino acid is glycine and ZAG-type complexes wherein the zirconium salt is $ZrO(OH)_{2-a}Cl_a \cdot nH_2O$ with a ranging from 1.5 to 1.87 and n ranging from 1 to 7; the aluminum salt is $Al_2(OH)_5Cl \cdot 2H_2O$; and the amino acid is glycine.

As indicated previously the present compositions contain from 10 % to 70 %, preferably from 15 % to 60 %, by weight of the particulate astringent antiperspirant materials calculated on an anhydrous metal salt basis (exclusive of glycine, the salts of glycine or other complexing agents). Such particulate antiperspirant material is preferably impalpable, i.e. has particle sizes ranging from 1 to 100 μm (microns), more preferably from 1 to 50 μm (microns).

Another essential component of the present compositions is a bulking or suspending agent. Such an agent is present at a level of from 1 % to 15 %, preferably 2 % to 8 %.

Clays and colloidal pyrogenic silica pigments and mixtures thereof, are the preferred materials for use as bulking/suspending agents. Colloidal silica is available commercially as Cab-O-Sil®, a submicroscopic particulated pyrogenic silica.

Clay bulking/suspending agents suitable for use in the compositions of the present invention are selected from montmorillonite clays and hydrophobically treated montmorillonite clays. Montmorillonite clays are those which contain the mineral montmorillonite and are characterized by having a suspending lattice. Examples of these clays include the bentonites, hectorites, and colloidal magnesium aluminum silicates.

Bentonite is colloidal, hydrated aluminum silicate obtained from montmorillonite and has the formula $Al_2O_34SiO_2 \cdot H_2O$. A more detailed discussion of bentonites can be found in the Kirk-Othmer Encyclopedia of chemical Technology, 2nd. ed., Vol. 3 (1964), pp. 339-360, published by Interscience Publishers. Hectorite, also a montmorillonite clay, differs from bentonite in that there is almost a complete substitution of aluminum in the lattice structure of bentonite by magnesium. In addition, hectorites contain lithium and fluorine. Laponite is an example of a commercially available synthetic hectorite marketed by Laporte, Industries, Ltd.

The magnesium aluminum silicates are

complexes of colloidal magnesium aluminum silicate richer in magnesium that aluminum. Magnesium aluminum silicates are commercially available as Veegum (R. T. Vanderbilt Co.).

Preferred clay suspending agents for use in the present invention are certain hydrophobically treated montmorillonite clays, e.g., hydrophobic bentonites available under the tradename of "Bentone". Bentone is prepared by reacting bentonite in a cation exchange system with an amine. Different amines are reacted to obtain a variety of Bentones, which may also differ in proportions of $SiO_2$, $M_gO$ and $Al_2O$. Specific examples of Bentones within the scope of the present invention are Bentone 38, Bentone 34, Bentone 27, Bentone 14, and Bentone LT, all of which have a particle size of below about 5 microns and are commercially available from the NL Industries, Inc.

A cyclic or a linear polydimethylsiloxane is present at a level of from 10 % to 80 %, preferably 15 % to 70 % by weight of the composition.

The cyclic polydimethylsiloxanes have 3 to 6 silicon atoms, preferably 5.

The general formula for such polydimethylsiloxanes is

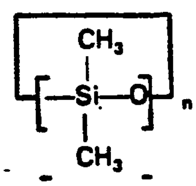

wherein n=3-6

The linear polydimethylsiloxanes have from 3 to 9 silicon atoms and have the general formula

$$(CH_3)_3Si\text{-}O\text{-}[\text{-}Si(CH_3)_2\text{-}O\text{-}]\text{-}_nSi(CH_3)_3$$

n = 1 - 7

Polydimethylsiloxanes of the above type are offered by Dow Corning Corporation, Dow Corning 344, 345 and 200 fluids, Union Carbide, Silicone 7207 and Silicone 7158, and Stauffer Chemical, SWS-03314.

The linear materials have viscosities of less than 5 $mm^2s^{-1}$ (cSt) at 25° C while the cyclic materials have viscosities less than 10 $mm^2s^{-1}$ (cSt). A description of volatile silicones is found in Todd and Byers, "Volatile Silicone Fluids for Cosmetics" Cosmetics and Toiletries, Vol. 91, January 1976, pp. 27-32.

The non-volatile emollient used in the present compositions is a silicone having a viscosity of from 10 to 100,000 $mm^2s^{-1}$ (cSt) at 25° C.

The non-volatile silicone fluid may be either a polyalkyl siloxane, a polyalkylaryl siloxane or a polyether siloxane copolymer.

The polyalkyl siloxanes that may be used include, for example, polydimethyl siloxanes with viscosities ranging from 10 to 100,000 $mm^2s^{-1}$ (cSt) at 25° C. These siloxanes are available, for example, from the General Electric Company as the Vicasil series and from Dow Corning as the Dow Corning 200 series.

The polyalkylaryl siloxanes that may be used include, for example, polymethylphenylsiloxanes having viscosities of 15 to 65 $mm^2s^{-1}$ (cSt) at 25° C. These siloxanes are available for example, from the General Electric Company as SF 1075 methyl phenyl fluid or from Dow Corning as 556 Cosmetic Grade Fluid.

The polyether siloxane copolymer that may be used is, for example, a dimethyl polyoxyalkylene ether copolymer fluid having a nominal viscosity of 1200 to 1500 $mm^2s^{-1}$ (cSt) at 25° C. This copolymer is available, for example, from the General Electric Company as SF-1066 organosilicone surfactant. Preferred compounds of this type are polysiloxane ethylene glycol ether copolymers.

In the present compositions, a ratio of non-volatile emollient to suspending agent of less than 10 : 1 is preferred when the suspending agent is a silica. The preferred ratio is less than 2: 1 when the suspending agent is a clay.

The optional components which may be used with the present compositions vary with the type of dispenser used.

If the dispenser of choice is an aerosol, the present compositions are combined with an aerosol propellant and perhaps a material to serve as carrier liquid. The propellant gas can be any liquefiable gas conventionally used for aerosol containers. Examples of materials that are suitable for use as propellants are trichlorofluoromethane, dichlorofluoromethane, dichlorotetrafluoroethane, monochlorodifluoromethane, trichlorotrifluoroethane, dimethylether, propane, butane and isobutane, used singly or admixed. Isobutane, used singly or admixed with other hydrocarbons, is preferred.

The amount of the propellant gas is governed by normal factors as well known in the aerosol art. The composition described previously herein serves as the concentrate and comprises from 7 % to 45 %, preferably 20 % to 40 % of the present total aerosol composition while the propellant comprises from 55 % to 93 %, preferably from 60 % to 80 %.

If a propellant such as dimethylether utilizes a vapor pressure suppressant (e.g. trichloroethane or dichloromethane) the amount of suppressant is included as part of the propellant.

Although the nonvolatile silicone may suitably serve as a carrier liquid in aerosols additional materials may also be used. The carrier liquid aids efficacy by keeping the antiperspirant compound in contact with the skin so that it does not flake off or wash off. Examples of additional materials are carboxylic esters like isopropyl myristate and isopropyl palmitate; alcohols such as lauryl alcohol, hexadecyl alcohol, and oleyl alcohol; carboxylic acids such as lauric and oleic acid; and lanolin and its derivatives such as

acetylated lanolin. Other operable carrier liquids are more hydrophilic than the above-mentioned compounds, for example, organic compounds containing multiple ester groups. This includes, but is not limited to, diesters of dibasic organic acids. Examples of compounds containing multiple ester groups that are suitable for the instant invention are di-n-butyl phthalate, diethyl sebacate, diisopropyl adipate, and ethyl ethoxycarbonylmethyl phthalate [ortho $C_2H_5OOC$-Ø-$COOCH_2COOC_2H_5$].

Still other operable carrier liquids are even more hydrophilic than these esters. Among them are polyethylene glycol monolaurate and butoxy-polyoxyethylene oxypropylene glycols (the Ucon 50 HB series; trade mark-Union Carbide).

Among these various carrier liquids, carboxylic esters having from 12 to 16 carbon atoms are preferred. As described supra, they can be either aliphatic or aromatic and can contain either one ester group or multiple ester groups. Especially preferred are di-n-butyl phthalate, diethyl sebacate, diisopropyl adipate, isopropyl myristate and ethyl ethoxycarbonylmethyl phthalate.

Any of the additional carrier liquids described supra can be used in amounts from 1 % to 15 % of the total aerosol composition.

The present compositions in aerosol form may also contain low levels of high molecular weight polymers similar to those described in US-A-4 152 416. These polymeric materials are used at a level of from 0.005 % to 5 % of the total aerosol composition. A preferred material is polyvinylisobutyl ether.

Another optional material in aerosol compositions is a polar material such as ethanol or propylene carbonate at a level of from 0.25 % to 5 % of the total aerosol composition.

If the present composition is used in a roll-on dispenser, a component such as ethanol may be present in an amount from 7 % to 18 %.

Regardless of the dispensing device employed, additional components such as perfumes, antimicrobials, fillers (e.g. talc) may be included in the compositions. If present these components comprise from 0.002 % to 10.0 % of the total compositions.

The compositions of the present invention are prepared by simply mixing together in any order and by conventional means known in the art the essential and optional components herein.

The present antiperspirant compositions are used in conventional manner.

Following are non-limiting examples of the present invention. All percentages in the examples and elsewhere herein are by weight unless otherwise specified.

**Example I**

A composition of the present invention having the following composition was formulated.

| | |
|---|---|
| Cyclomethicone[1] | 58.20 % |
| Cab-o-Sil HS-5[2] (fumed silica) | 3.50 |
| ZAG[3] | 26.70 |
| Dimethicone[4]350 ($mm^2s^{-1}$ (cSt) Viscosity at 25°C | 11.60 |
| | 100.00 % |

[1] Volatile cyclic polydimethylsiloxane having 5 silicon atoms offered by Union Carbide Corporation-Silicone 7158.
[2] Cabot Corporation
[3] Complex of zirconyl hydroxychloride as taught in US-A-3 792 068.
[4] Non-volatile polydimethyl silicone offered by Dow Corning-Dow Corning 200.

The above composition is used in a dispenser such as that described in US-A-4 167 245, and is a very effective antiperspirant.

When Cab-o-Sil HS-5 is replaced by a Bentone a composition having similar improved efficacy is achieved. Similarly the cyclomethicone may be replaced by another volatile polydimethylsiloxane and the dimethicone may be replaced by another nonvolatile silicone.

**Example II**

A composition of the present invention is as follows:

| | |
|---|---|
| Cyclomethicone | 7.000 % |
| Bentone 38 | 1.250 |
| Isopropyl Myristate | 7.145 |
| Propylene Carbonate | 0.400 |
| Aluminum chlorohydroxide ($Al_2(OH)_5Cl \cdot 2H_2O$) | 12.000 |
| Ethylene Brassylate | 0.005 |
| Dimethicone 60,000 $mm^2s^{-1}$ (cSt) viscosity at 25°C | 3.000 |
| Isobutane | 69.200 |
| | 100.000 % |

The above composition is used in a conventional aerosol container.

**Claims**

1. An antiperspirant composition in the form of a suspension comprising:

(a) from 10 % to 70 % by weight of a particulate antiperspirant material;

(b) from 1 % to 15 % by weight of a bulking/suspending agent; and

(c) from 10 % to 80 % by weight of a cyclic polydimethylsiloxane having from 3 to 6 silicon atoms and a viscosity of less than 10$mm^2s^{-1}$ (cSt) at 25°C or a linear polydimethylsiloxane having

from 3 to 9 silicon atoms and a viscosity of less than 5 $mm^2s^{-1}$ (cSt) at 25° C;

characterized in that the composition additionally comprises:

(d) from 1 % to 35 % by weight of a non-volatile emollient selected from silicones having a viscosity of from 10 to 100,000 $mm^2s^{-1}$ (cSt) at 25° C.

2. An antiperspirant composition according to Claim 1 characterized in that the bulking/suspending agent is selected from clays and colloidal pyrogenic silica pigments and mixtures thereof.

3. An antiperspirant composition according to any of Claims 1 to 2 characterized in that the antiperspirant material is an aluminium salt.

4. An antiperspirant composition according to any of Claims 1 to 3 characterized in that the particulate antiperspirant material is a zirconium aluminum complex.

5. An antiperspirant composition according to any preceding Claim characterized in that the amount of particulate antiperspirant material is from 15 % to 60 % by weight, the amount of bulking/suspending agent is from 2 % to 8 % by weight, the amount of cyclic or linear polydimethylsiloxane is from 15 % to 70 % by weight and the amount of non-volatile emollient is from 5 % to 30 % by weight.

6. An antiperspirant composition according to any preceding Claim characterized in that the polydimethylsiloxane is cyclic.

7. An antiperspirant composition according to any preceding Claim characterized in that the particulate antiperspirant material is an aluminium zirconium glycine complex.

8. An antiperspirant composition according to any preceding Claim characterized in that the non-volatile emollient is a polydimethyl siloxane.

9. An aerosol antiperspirant composition characterized by from 7 % to 45 % by weight of a composition according to any of Claims 1 to 8 and from 55 % to 93 % by weight of an aerosol propellant.

## Patentansprüche

1. Eine Antitranspirant-Zusammensetzung in der Form einer Suspension, umfassend:

(a) 10 Gew.-% bis 70 Gew.-% eines teilchenförmigen Antitranspirant-Materials;

(b) 1 Gew.-% bis 15 Gew.-% eines Streck/Suspendiermittels; und

(c) 10 Gew.-% bis 80 Gew.-% eines cyclischen Polydimethylsiloxans mit 3 bis 6 Siliciumatomen und einer Viskosität von weniger als 10 $mm^2s^{-1}$ (cSt) bei 25° C, oder eines linearen Polydimethylsiloxans mit 3 bis 9 Siliciumatomen und einer Viskosität von weniger als 5 $mm^2s^{-1}$ (cSt) bei 25° C;

dadurch gekennzeichnet, daß die Zusammensetzung zusätzlich:

(d) 1 Gew.-% bis 35 Gew.-% eines nichtflüchtigen Erweichungsmittels, ausgewählt aus Silikonen mit einer Viskosität von 10 bis 100.000 $mm^2s^{-1}$ (cSt) bei 25° C, enthält.

2. Eine Antitranspirant-Zusammensetzung gemäß Anspruch 1, dadurch gekennzeichnet, daß das Streck/Suspendiermittel aus Tonen und kolloidalen, pyrogenen Kieselsäurepigmenten, und Mischungen davon, ausgewählt ist.

3. Eine Antitranspirant-Zusammensetzung gemäß irgendeinem der Ansprüche 1 bis 2, dadurch gekennzeichnet, daß das Antitranspirant-Material ein Aluminiumsalz ist.

4. Eine Antitranspirant-Zusammensetzung gemäß irgendeinem der Ansprüche 1 bis 3, dadurch gekennzeichnet, daß das teilchenförmige Antitranspirant-Material ein Zirkonaluminiumkomplex ist.

5. Eine Antitranspirant-Zusammensetzung nach irgendeinem vorhergehenden Anspruch, dadurch gekennzeichnet, daß die Menge an teilchenförmigem Antitranspirant-Material 15 Gew.-% bis 60 Gew.-%, die Menge an Streck/Suspendiermittel 2 Gew.-% bis 8 Gew.-%, die Menge an cyclischem oder linearem Polydimethylsiloxan 15 Gew.-% bis 70 Gew.-%, und die Menge an nicht-flüchtigem Erweichungsmittel 5 Gew.-% bis 30 Gew.-% beträgt.

6. Eine Antitranspirant-Zusammensetzung nach irgendeinem vorhergehenden Anspruch, dadurch gekennzeichnet, daß das Polydimethylsiloxan cyclisch ist.

7. Eine Antitranspirant-Zusammensetzung nach irgendeinem vorhergehenden Anspruch, dadurch gekennzeichnet, daß das teilchenförmige Antitranspirant-Material ein Aluminiumzirkonglycinkomplex ist.

8. Eine Antitranspirant-Zusammensetzung nach irgendeinem vorhergehenden Anspruch, dadurch gekennzeichnet, daß das nicht-flüchtige Erweichungsmittel ein Polydimethylsiloxan ist.

9. Eine Aerosolantitranspirant-Zusammensetzung, gekennzeichnet durch 7 Gew.-% bis 45 Gew.-% einer Zusammensetzung nach irgendeinem der Ansprüche 1 bis 8 und 55 Gew.-% bis 93 Gew.-% eines Aerosoltreibmittels.

## Revendications

1. Une composition antiperspirante sous la forme d'une suspension comprenant:

(a) de 10 à 70 % en poids d'une matière antiperspirante particulaire;

(b) de 1 à 15 % en poids d'un agent de gonflement/mise en suspension; et

(c) le 10 à 80 % en poids d'un polydiméthylsiloxane cyclique ayant de 3 à 6 atomes de silicium et une viscosité inférieure à 10 $mm^2s^{-1}$ (cSt) à 25° C, ou d'un polydiméthylsilosane linéaire ayant de 3 à 9

atomes de carbone et une viscosité inférieure à 5 $mm^2s^{-1}$ (cSt) à 25° C; caractérisée en ce que la composition comprend en outre:

(d) de 1 à 35 % en poids d'un émollient non-volatil choisi parmi les silicones ayant une viscosité de 10 à 100.000 $mm^2s^{-1}$ (cSt) à 25° C.

2. Une composition antiperspirante selon la revendication 1, caractérisée en ce que l'agent de gonflement/de mise en suspension est choisi parmi les argiles et les pigments de silice pyrogène colloïdale et leurs mélanges.

3. Une composition antiperspirante selon l'une quelconque des revendications 1 et 2, caractérisée en ce que la matière antiperspirante est un sel d'aluminium.

4. Une composition antiperspirante selon l'une quelconque des revendications 1 à 3, caractérisée en ce que la matière antiperspirante particulaire est un complexe d'aluminium et de zirconium.

5. Une composition antiperspirante selon l'une quelconque des revendications précédentes, caractérisé en ce que la quantité de la matière antiperspirante particulaire est de 15 à 60 % en poids, la quantité de l'agent de gonflement / de mise en suspension est de 2 à 8 % en poids, la quantité, de polydiméthylsiloxane cyclique ou linéaire est de 15 à 70 % en poids et la quantité de l'émollient non volatil est de 5 à 30 % en poids.

6. Une composition antiperspirante selon l'une quelconque des revendications précédentes, caractérisée en ce que le polydiméthylsiloxane est cyclique.

7. Une composition antiperspirante selon l'une quelconque des revendications précédentes, caractérisée en ce que la matière antiperspirante particulaire est un complexe de glycine, de zirconium et d'aluminium.

8. Une composition antiperspirante selon l'une quelconque des revendications précédentes, caractérisée en ce que l'émollient non-volatil est un polydiméthylsiloxane.

9. Une composition antiperspirante en aérosol ceractérisée par 7 à 45 % en poids d'une composition selon l'une quelconque des revendications 1 à 8 et de 55 à 93 % en poids d'un propulseur pour aérosol.